# EUROPEAN PATENT SPECIFICATION

(11) **EP 2 502 545 B1**
(45) Date of publication and mention of the grant of the patent: **14.05.2014**
(21) Application number: 11765245.3
(22) Date of filing: 05.01.2011
(51) Int. Cl.: A61B 1/00, A61B 19/02, B65D 81/07

(54) **PACKAGE FOR CAPSULE MEDICAL DEVICE**
VERPACKUNG FÜR EINE MEDIZINISCHE KAPSELVORRICHTUNG
EMBALLAGE POUR DISPOSITIF MEDICAL A CAPSULE

(30) Priority: 05.04.2010 JP 2010087276
(43) Date of publication of application: 26.09.2012
(73) Proprietor: OLYMPUS MEDICAL SYSTEMS CORP., Tokyo 151-0072 (JP)
(72) Inventor: TAKAHASHI, Masaki, Tokyo 151-0072 (JP); SEGAWA, Hidetake, Tokyo 151-0072 (JP)
(74) Representative: Schicker, Silvia
(86) International application number: PCT/JP2011/050054
(87) International publication number: WO 2011/125343

(56) References cited:
- EP-A2- 1 518 795
- WO-A1-2007/074883
- WO-A1-2008/053893
- JP-A- 7 165 260
- JP-A- 8 501 046
- JP-A- 2005 073 884
- JP-A- 2005 095 433
- JP-A- 2005 278 815
- JP-A- 2006 087 524
- JP-A- 2006 288 743
- JP-A- 2006 314 808
- JP-A- 2007 068 761
- JP-A- 2007 159 866
- JP-A- 2009 195 558
- JP-U- H0 390 868
- JP-U- 63 137 769
- US-A1- 2008 039 675
- US-A1- 2008 103 372
- US-B1- 6 499 599

## Description

### Field

The present invention relates to a package for a capsule medical device, the package retaining a capsule medical device.

### Background

In recent years, a capsule medical device (capsule endoscope) provided with an imaging function and a radio communication function has made an appearance in the field of an endoscope. The capsule endoscope, after being swallowed by an examinee as a subject for the purpose of an observation (examination), travels an inside of organs such as a stomach and a small intestine (inside of a body cavity) according to their peristalsis during an observation period which ends when it is naturally excreted from a living body of the examinee, and sequentially captures images by using the imaging function.

Image data captured in the inside of the body cavity by the capsule endoscope during the observation period through the traveling in the inside of the organs is sequentially transmitted to an external device provided at an outside of the subject by the radio communication function like a radio communication and stored in a memory provided in the external device. The examinee, by carrying the external device provided with the radio function and the memory function, is able to move without any inconvenience during the observation period which starts when the capsule endoscope is swallowed and ends when excreted. After the observation, a doctor or a nurse is able to have the images of the inside of the body cavity displayed on a displaying unit such as a display device based on the image data stored in the memory of the external device to make a diagnosis.

Incidentally, since it is necessary to sterilize the capsule endoscope prior to a use on the subject, a process of sterilizing the capsule endoscope is performed by injecting sterilizing gas, while keeping the capsule endoscope housed in a package, into the package. As the package, a package has been proposed in which the capsule endoscope is inserted along its long axis direction into a hole formed on a main surface of a base container in such a manner that a body of the capsule endoscope is buried and then the main surface of the base container is sealed with a sterilization sheet (see Patent Literature 1).

### Citation List

### Patent Literature

Patent Literature 1: Japanese Patent Application Laid-Open No. 2006-87524 US 2008/0103372 A1 discloses a container case for a capsule medical device. The container case comprises an outer blister pack and an inner lid unit.

JP 2005/073884 discloses an enclosure member formed integrally with a shield member so as to form a protection cap holding a pill.

JP 2006/087524 A discloses a capsule endoscope retained by a retaining part in a line contact. The retaining part is housed in a main body of a housing part of the housing case, and the opening of the main body is subjected to heat sealing by a sterilizing sheet with sterilizing gas permeability.

US 2008/0039675 A1 discloses a capsule endoscope storage package.

### Summary

### Technical Problem

However, the conventional package has a problem in that, because the body of the capsule endoscope is deeply fitted therein, less part can be picked up with fingers and therefore the capsule endoscope cannot be easily taken out from the package. The conventional package also has a problem in that, when the capsule endoscope is taken out from the package, the transparent dome part of the casing of the capsule endoscope, which is the transparent part at the end covering the imaging unit, may be touched and thus have a dirt, which reduces the observation quality.

The present invention was made in view of the above-described circumstances. An object of the present invention is to provide a package for a capsule medical device that makes it possible to take out the capsule medical device easily without touching the transparent dome part of the casing of the capsule medical device, which is the transparent dome part at the end covering the imaging unit.

### Solution to Problem

The present invention is defined in claim 1.

In order to solve the above-described problems and to achieve the above-described object, a package for a capsule medical device according to the present invention is a package for a capsule medical device which retains a capsule medical device, the package including: a plurality of retainers that retain a body of the capsule medical device in a manner of allowing the capsule medical device to be taken out from the package; and a non-retaining part that is formed between the retainers and that has the body of the capsule medical device exposed allowing grasp of the body of capsule medical device, wherein the retainers and the non-retaining part guide the grasp of the body of the capsule medical device from an outside.

A package for a capsule medical device includes: a grasp preventive part that is formed in a manner of covering at least one of the ends of the capsule medical device in a long axis and prevents at least the one of the ends of the capsule medical device in the long axis from being grasped.

In a package for a capsule medical device, the retainers retain respective edge parts of the body of the capsule medical device in such a manner that the capsule medical device can be taken out from the package in a diametrical direction of the capsule medical device.

In a package for a capsule medical device, the retainers are provided at both edge parts of the body of the capsule medical device and retain the edge parts of the body of the capsule medical device.

In a package for a capsule medical device, each of the retainers includes a pair of contact parts which are in contact with the edge part of the body of the capsule medical device, the contact parts provided in pairs are formed to sandwich the body of the capsule medical device therebetween, and a distance between the contact parts provided in pairs is shorter than an outer diameter of the body of the capsule medical device.

In a package for a capsule medical device, each of the retainers includes a protruding part protruding to prevent a separation of the capsule medical device in the diametrical direction and a distance between facing surfaces of the protruding parts of the retainers is shorter than an outer diameter of the body of the capsule medical device.

The package for a capsule medical device according to the present invention further includes a regulator that regulates a movement of the capsule medical device along a plane perpendicular to a direction along which the capsule medical device housed in the package is taken out.

In a package for a capsule medical device, each of the retainers includes a pair of contact parts which are in contact with the edge part of the body of the capsule medical device and a pressure receiving part, the contact parts provided in pairs are framed to sandwich the body of the capsule medical device therebetween, a distance between the contact parts provided in pairs is shorter than an outer diameter of the body of the capsule medical device, and the pressure receiving part is formed integrally with the contact parts provided in pairs and allows the distance between the contact parts provided in pairs to be widened to be equivalent to or more than the outer diameter of the body of the capsule medical device when depressed from a predetermined depression direction.

In a package for a capsule medical device, the retainers retain the capsule medical device housed in the package in one of a point contact manner and a line contact manner.

In a package for a capsule medical device, the retainers retain at least one part of an edge part of the body of the capsule medical device in such a manner that the capsule medical device can be taken out from the package in a longitudinal direction of the capsule medical device.

The package for a capsule medical device according to the present invention further includes: a base part in which a hole into which a predetermined end of the capsule medical device in a long axis is fitted and mounted is formed on a main surface, wherein the retainers are formed by a convex part protruding from the main surface of the base part uninterruptedly with a part of a side wall of the hole so that a part of the body of the capsule medical device is exposed to allow the grasp.

In a package for a capsule medical device information indicating a grasp direction is shown on the main surface of the base part.

A package for a capsule medical device includes: a base part in which a hole into which a predetermined end of the capsule medical device in a long axis and the body of the capsule medical device are fitted and mounted is formed on a main surface; and a concave part formed in such a manner that a part of the main surface of the base part is dug down uninterruptedly with a part of a side wall of the hole, wherein the retainers are formed by a part except for the concave part on the main surface of the base part.

In a package for a capsule medical device, a surface of the package retaining the capsule medical device is closed by a sterilization sheet having sterilizing gas permeability. Advantageous Effects of Invention

A package for a capsule medical device according to the present invention includes retainers that retain at least a part of a body of the capsule medical device in a manner of allowing the capsule medical device to be taken out from the package; and a non-retaining part that is formed between the retainers and that has the body of the capsule medical device exposed allowing grasp of the body of capsule medical device, wherein the retainers and the non-retaining part guide the grasp of the body of the capsule medical device from an outside. Accordingly, it is possible to take out the capsule medical device easily without touching a transparent dome part of a casing of the capsule medical device.

### Brief Description of Drawings

FIG. 1 is a conceptual system diagram showing a concept of a capsule endoscope system according to a first example useful for understanding the invention;
FIG. 2 is a cross sectional view showing a schematic diagram of a configuration example of the capsule endoscope 2 shown in FIG. 1;
FIG. 3 is a perspective view of a configuration of a package for a capsule endoscope according to the first example useful for understanding the invention;
FIG. 4 is a perspective view of one example of a case where a sterilization sheet is removed from the package shown in FIG. 3;
FIG. 5 is a top view of an upper surface of the base container shown in FIG. 4;
FIG. 6 is a cross sectional view along the line AA in FIG. 5;
FIG. 7 is a perspective view of a base container that forms a package for a capsule endoscope according to a second example useful for understanding the invention;
FIG. 8 is a top view of an upper surface of the base container shown in FIG. 7;
FIGS. 9 includes cross sectional views along the line BB in FIG. 8;
FIG. 10 is a perspective view of a base container that forms a package for a capsule endoscope according to a first modification of the second example useful for understanding the invention;
FIGS. 11 includes cross sectional views along the line CC in FIG. 10;
FIG. 12 is a cross sectional view along the line DD in FIG. 10;
FIG. 13 is a cross sectional view along the line EE in FIG. 12;
FIG. 14 is a perspective view of a base container that forms a package for a capsule endoscope according to a third example useful for understanding the invention;
FIG. 15 is a top view of an upper surface of the base container shown in FIG. 14;
FIGS. 16 includes cross sectional views along the line FF in FIG. 15;
FIGS. 17 includes cross sectional views of another example of the base container according to the third example useful for understanding the invention;
FIG. 18 is a perspective view of a configuration of a package for a capsule endoscope according to a fourth example and embodiment of the invention;
FIG. 19 is a perspective view of one example of a case where the sterilization sheet is removed from the package shown in FIG. 18;
FIG. 20 is a perspective view of the base container shown in FIG. 19;
FIG. 21 is a top view of an upper surface of the base container shown in FIG. 20;
FIG. 22 is a cross sectional view along the line GG in FIG. 21;
FIG. 23 shows another example of the cross sectional view along the line GG in FIG. 21;
FIG. 24 is a top view of another example of the base container shown in FIG. 19;
FIG. 25 is a top view of still another example of the base container shown in FIG. 19;
FIG. 26 is a top view of still another example of the base container shown in FIG. 19;
FIG. 27 is a perspective view of a base container that forms a package for a capsule endoscope according to a fifth example useful for understanding the invention;
FIG. 28 is a top view of an upper surface of the base container shown in FIG. 27; and
FIG. 29 is a cross sectional view along the line HH in FIG. 28.

### Description of Examples and Embodiments

Examples of a package for a capsule medical device according to the present invention will be explained in detail below with reference to the accompanying drawings. It should be noted that the present invention is not limited to the embodiments. The same part is assigned with the same reference symbol in the description of the drawings. It should also be noted that the accompanying drawings are merely schematic and a relation between a thickness and a width of each member, a ratio of each member, and the like may be different from the reality. The dimensional relations and the ratio may be different from one drawing to another.

### (First example useful for understanding the invention)

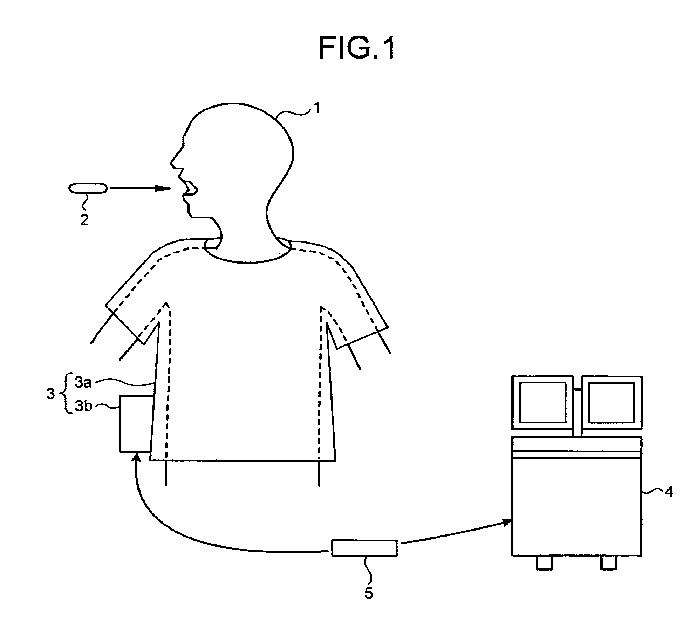

FIG. 1 is a conceptual system diagram showing a concept of a capsule endoscope system. In FIG. 1, a capsule endoscope system is provided with a swallowable capsule endoscope 2 serving as a capsule medical device that is inserted to the inside of the body cavity of a subject 1 to capture and wirelessly transmit images of the inside of the subject, and a receiving device 3 serving as an extracorporeal device that is arranged at the outside of the subject 1 and performs a radio communication on information of various kind with the capsule endoscope 2. The in-vivo information acquiring system of radio type is provided with a display device 4 that displays images based on data the receiving device 3 has received and a portable recording medium 5 that allows inputting and outputting data between the receiving device 3 and the display device 4.

The receiving device 3 serves as a radio receiving unit that receives data of in-vivo images of the subject 1 wirelessly transmitted from the capsule endoscope 2. The receiving device 3 is provided with a receiving jacket 3a which is worn by the subject 1 and includes a plurality of receiving antennas not shown, and an external device 3b which performs a signal process on the received radio signal.

The display device 4 displays intra-subject images captured by the capsule endoscope 2 and has a configuration such as a workstation in which images are displayed based on the data obtained by the portable recording medium 5. Specifically, the display device 4 may be configured to directly display the images by a CRT display device, a liquid crystal display device, and the like, or may be configured to output the images to another medium such as a printer.

The portable recording medium 5 can be connected to the external device 3b and the display device 4 and has a structure which allows the information to be output or recorded when attached and connected to both devices. In the present example, the portable recording medium 5 is inserted into the external device 3b to record the data transmitted from the capsule endoscope 2 while the capsule endoscope 2 moves in the inside of the subject 1. After the capsule endoscope 2 is excreted from the subject 1, namely, after the imaging in the subject 1 is finished, the portable recording medium 5 is then taken out from the external device 3b and inserted into the display device 4 and the data recorded in the portable recording medium 5 is read by the display device 4. For example, the portable recording medium 5 is configured by CompactFlash (registered trademark) memory and the like, the data can indirectly be input and output between the external device 3b and the display device 4 through the portable recording medium 5, and the subject 1 is able to freely move during the imaging unlike the case where the external device 3b and the display device 4 are directly connected by a cable.

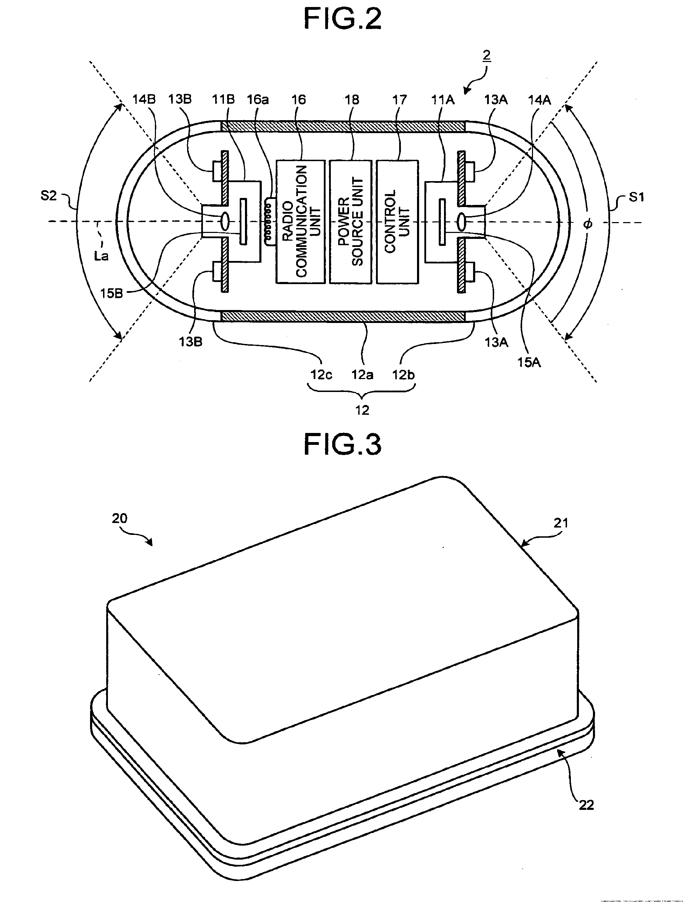

Next, the capsule endoscope 2 shown in FIG. 1 will be explained. FIG. 2 is a cross sectional view showing a schematic diagram of a configuration example of the capsule endoscope 2 shown in FIG. 1. As shown in FIG. 2, the capsule endoscope 2 is provided with a capsule-shaped casing 12 which is an exterior formed in a size allowing an easy insertion into the inside of organs of the subject and imaging units 11A and 11B that capture images of the subject in respective imaging directions different to each other. The capsule endoscope 2 is provided with a radio communication unit 16 that wirelessly transmits each of the images captured by the imaging units 11A and 11B to the outside via an antenna 16a, a control unit 17 that controls each component of the capsule endoscope 2, and a power source unit 18 that supplies power to each component of the capsule endoscope 2.

The capsule-shaped casing 12 is an outer casing formed in a size allowing an insertion to the inside of organs of the subject and realized by blocking open edges at both sides of a cylindrical casing 12a with dome-shaped casings 12b and 12c. The dome-shaped casings 12b and 12c are optical members which have a dome like shape and a transparency with respect to a light of a predetermined wavelength band such as a visible light. The cylindrical casing 12a is a colored casing which is nearly opaque with respect to the visible light. The capsule-shaped casing 12 formed by the cylindrical casing 12a and the dome-shaped casings 12b and 12c includes therein the imaging units 11A and 11B, the radio communication unit 16, the control unit 17, and the power source unit 18 in a liquid-tight manner.

The imaging units 11A and 11B capture images in respective imaging directions different to each other. Specifically, the imaging unit 11A includes an illumination unit 13A such as an LED, an optical system 14A such as a condenser lens, and an imaging element 15A such as a CMOS image sensor or a CCD. The illumination unit 13A emits an illumination light such as a white color light to an imaging field S1 of the imaging element 15A to illuminate the subject within the imaging field S1 (inner wall of organs at a side of the imaging field S1 in the inside of the subject, for example) through the dome-shaped casing 12b. The optical system 14A condenses a reflection light from the imaging field S1 on an imaging surface of the imaging element 15A to form a subject image in the imaging field S1 on the imaging surface of the imaging element 15A. The imaging element 15A receives the reflection light from the imaging field S1 via the imaging surface and performs a photoelectric conversion process with respect to the received optical signal to capture the subject image in the imaging filed S1 (angle of field ϕ), i.e., the in-vivo image of the subject. The imaging unit 11B includes an illumination unit 13B such as an LED, an optical system 14B such as a condenser lens, and an imaging element 15B such as a CMOS image sensor or a CCD. The illumination unit 13B emits an illumination light such as a white color light to an imaging field S2 of the imaging element 15B to illuminate the subject within the imaging field S2 (inner wall of organs at a side of the imaging field S2 in the inside of the subject, for example) through the dome-shaped casing 12c. The optical system 14B condenses a reflection light from the imaging field S2 on an imaging surface of the imaging element 15B to form a subject image in the imaging field S2 on the imaging surface of the imaging element 15B. The imaging element 15B receives the reflection light from the imaging field S2 via the imaging surface and performs a photoelectric conversion process with respect to the received optical signal to capture the subject image in the imaging filed S2 (angle of field ϕ), i.e., the in-vivo image of the subject.

When the capsule endoscope 2 is a binocular-type capsule medical device that captures respective images of the front and the rear in a direction along a long axis La as shown in FIG. 2, optical axes of the imaging units 11A and 11B are nearly parallel with or nearly accord with the long axis La as a central axis in the longitudinal direction of the capsule-shaped casing 12. In addition, the directions of the imaging fields S1 and S2 of the imaging units 11A and 11B, i.e., the imaging directions of the imaging units 11A and 11B are opposite with each other.

The control unit 17 controls operations of the imaging units 11A and 11B and the radio communication unit 16 which are components of the capsule endoscope 2 and controls input and output of a signal among these components. Specifically, the control unit 17 controls the imaging element 15A to capture images of the subject in the imaging field S1 illuminated by the illumination unit 13A and the imaging element 15B to capture images of the subject in the imaging field S2 illuminated by the illumination unit 13B. Besides, the control unit 17 includes a signal processing function of generating an image signal. The control unit 17 obtains in-vivo image data in the imaging field S1 from the imaging element 15A and performs a predetermined signal process with respect to the in-vivo image data each time of the obtainment to generate an image signal containing the in-vivo image data in the imaging field S1. Similarly to this, the control unit 17 obtains in-vivo image data in the imaging field S2 from the imaging element 15B and performs a predetermined signal process with respect to the in-vivo image data each time of the obtainment to generate an image signal containing the in-vivo image data in the imaging field S2. The control unit 17 controls the radio communication unit 16 to wirelessly transmit each image signal sequentially to the outside along time series.

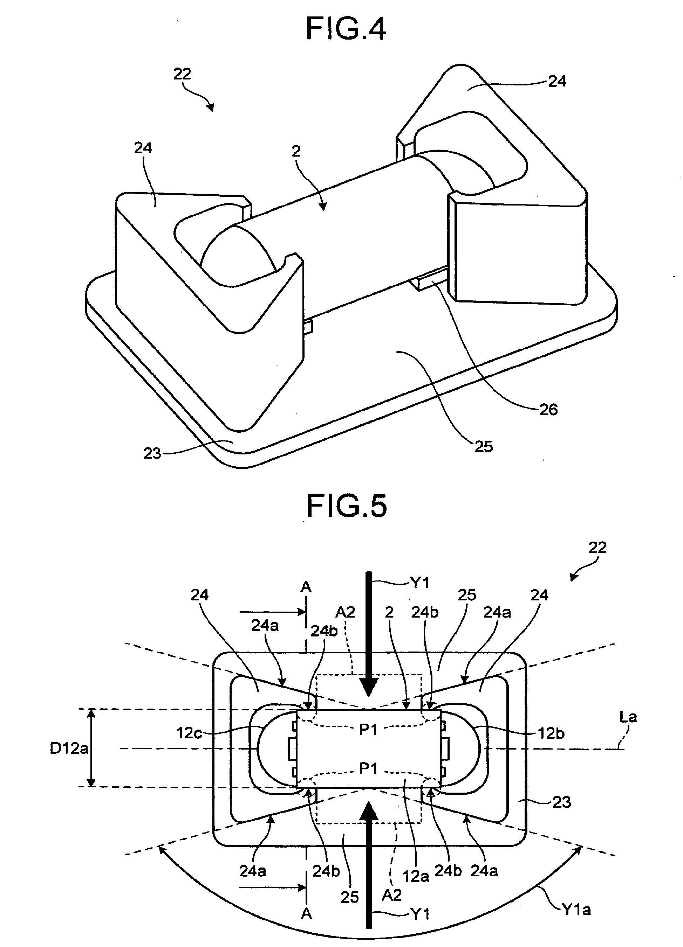

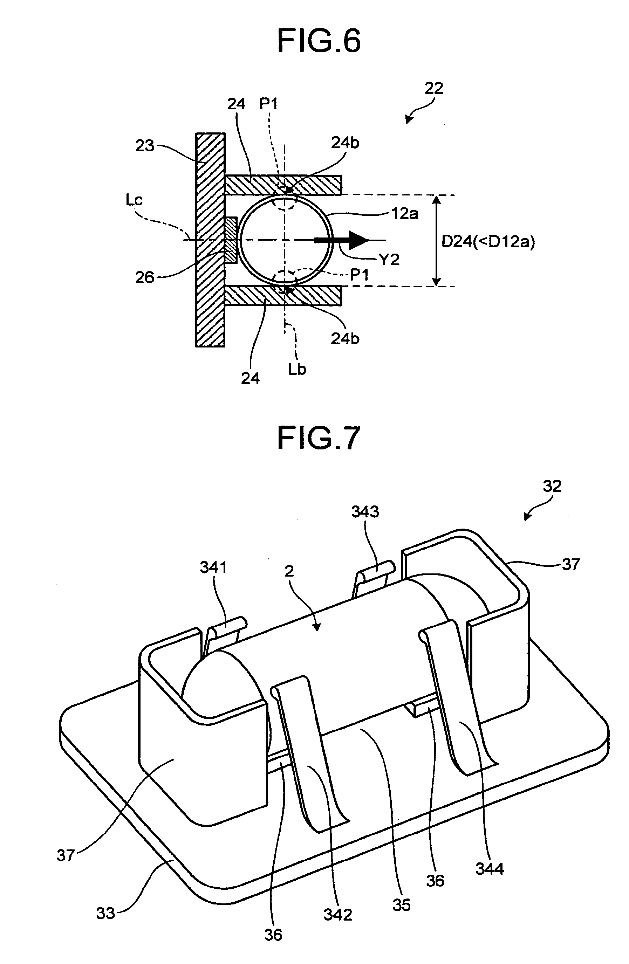

Incidentally, it is necessary to sterilize the capsule endoscope 2 prior to the use on the subject 1 and keep the sterilized state. In the first example, the capsule endoscope 2 is housed in a package which allows sterilization. The package for the capsule endoscope according to the first example will be explained below with reference to FIGS. 3 to 6. Here, FIG. 3 is a perspective view of a configuration of the package for the capsule endoscope according to the first example, the package storing the capsule endoscope, FIG. 4 is a perspective view of one example of a case where a sterilization sheet is removed from the package shown in FIG. 3, FIG. 5 is a top view of an upper surface of the base container shown in FIG. 4, and FIG. 6 is a cross sectional view along the line AA in FIG. 5.

As shown in FIG. 3, a package 20 for the capsule endoscope 2 according to the first example has a configuration in which a main surface of a base container 22 on which the capsule endoscope 2 can be retained is closed by a sterilization sheet 21 having sterilizing gas permeability. The sterilization sheet 21 forms a sealing member. A sterilizing process on the capsule endoscope 2 is performed by supplying sterilizing gas to an inside of the package 20 via the sterilization sheet 21 after the capsule endoscope 2 is retained in the base container 22 of the package 20 and then closed by the sterilization sheet 21.

As shown in FIGS. 4 to 6, two retainers 24 are formed in the base container 22 in a manner of protruding upward from an upper surface of a base member 23 along a vertical axis of the upper surface of the base member 23. The two retainers 24 are provided at both edges of a body of the capsule endoscope 2, i.e., at both edges of the cylindrical casing 12a to retain both edges of the body of the capsule endoscope 2. The two retainers 24 retain the body of the capsule endoscope 2 in such a manner that the capsule endoscope 2 can be taken out from the base container 22 in a diametrical direction of the capsule endoscope 2 (in a direction along an axis Lc perpendicular to the upper surface of the base member 23 shown in FIG. 6). Each of the retainers 24 has a structure of protruding from the upper surface of the base member 23 in the direction along which the capsule endoscope 2 is taken out.

The retainers 24 are formed to cover respective ends of the capsule endoscope 2 in the long axis La. As shown in FIG. 5, the retainers 24 are formed around the dome-shaped casings 12b and 12c in which the imaging units 11A and 11B of the capsule endoscope 2 are respectively included when seen in the top view. As shown in FIG. 6, the retainers 24 have a height almost equivalent to an outer diameter of the capsule endoscope 2 on the base member 23. Thus, the retainers 24 prevent both ends of the capsule endoscope 2 in the long axis from being grasped from the direction along the long axis La of the capsule endoscope 2.

As shown in FIGS. 5 and 6, each of the retainers 24 has a pair of contact parts 24b which are in contact with a part of an edge part of the body of the capsule endoscope 2. The pair of contact parts 24b of the retainer 24 is configured to mutually put the body of the capsule endoscope 2 therebetween. The retainer 24 sandwiches the edge part of the body of the capsule endoscope 2 by two facing surfaces on which respective contact parts 24b locate. In the example shown in FIG. 6, the pair of contact parts 24b and the edge part of the body of the capsule endoscope 2 are in line contact in a cross line between a parallel plane (a surface Lb shown in FIG. 6) which is parallel to the upper surface of the base member 23 and passes through the central axis of the capsule endoscope 2 and an inner wall of the retainer 24 as shown in an area P1. The width of the pair of contract parts 24b in the direction along the long axis La may be narrowed so that the edge part of the body of the capsule endoscope 2 and the respective contract parts 24b have point contact with each other in the retainer 24.

The retainer 24 is formed in such a manner that a distance D24 (see FIG. 6) along the surface Lb between the pair of contact parts 24b is smaller than an outer diameter of the body of the capsule endoscope 2, i.e., an outer diameter D12a (see FIG. 5) of the cylindrical casing 12a. Therefore, the pair of contact parts 24b supports the capsule endoscope 2 by a friction generated with the body of the capsule endoscope 2 so that the capsule endoscope 2 does not involuntarily jump out in the direction along which the capsule endoscope 2 is taken out (a direction along the axis Lc shown in FIG. 6). Besides, the retainer 24 is formed to support the capsule endoscope 2 with an adequate force so as to allow taking out of the capsule endoscope 2 by a grasp with fingers from the base container 22.

One retainer 24 supports the edge part of the body of the capsule endoscope 2 at two spots. Thus, the two retainers 24 are in contact with respective edge parts of the body of the capsule endoscope 2 at four spots, thereby retaining the capsule endoscope 2 in the base container 22. As shown in FIG. 6, a supporting plate 26 which supports the edge part of the body of the capsule endoscope 2 is provided on the upper surface of the base member 23 to reduce an area in which the body of the capsule endoscope 2 and the upper surface of the base member 23 are in contact.

The two retainers 24 are formed away from each other in a manner of allowing the body of the capsule endoscope 2 to be exposed. An area not occupied by the retainers 24 between the retainers 24 serves as a non-retaining part 25. In an area A2 in the non-retaining part 25 in FIG. 5, the body of the capsule endoscope 2 is exposed allowing the grasp.

As shown in FIG. 5, an inclination surface 24a as an outer wall of the retainer 24 is formed, when seen from the top, in a manner of being inclined with respect to the long axis La so that the retainer 24 is shaped to be widened from the edge part of the body of the capsule endoscope 2 toward the end of the capsule endoscope 2 in the long axis. Thus, the non-retaining part 25 has a form of spreading from the center of the body of the capsule endoscope 2 toward the long axis La as shown by an arrow Y1a. As a result of this, when two fingers of the operator approach from both sides in the diametrical direction of the capsule endoscope 2 like an arrow Y1 as shown in FIG. 5, the fingers are guided along the inclination surface 24a of the retainer 24 to the body of the capsule endoscope 2 exposed in the area A2. Thus, the retainer 24 and the non-retaining part 25 have the body of the capsule endoscope 2 exposed allowing the grasp and guide the grasp of the body of the capsule endoscope 2 from an outside not to grasp a part other than the body of the capsule endoscope 2. The operator, after the fingers are guided to the body of the capsule endoscope 2, grasps the body of the capsule endoscope 2 directly to raise and take out the capsule endoscope 2 along the axis Lc as shown by an arrow Y2 in FIG. 6 from the base container 22. When the base container 22 is seen from the top as shown in FIG. 5, the operator, after having fingers approach like the arrow Y1 and grasping the body of the capsule endoscope 2 with the fingers, takes out the capsule endoscope 2 upward in a nearly vertical direction to the paper plane.

In this manner, since the capsule endoscope 2 is retained in the state where the body of the capsule endoscope 2 is widely exposed, it is possible in the first embodiment to secure a wide area to be grasped by fingers and take out the capsule endoscope 2 with fingers easily from the base container 22.

In the first example, since the retainers 24 are formed around the dome-shaped casings 12b and 12c respectively including therein the imaging units 11A and 11B, the dome-shaped casings 12b and 12c are not grasped by fingers from the direction along the long axis La. Therefore, since the dome-shaped casings 12b and 12c do not have contact with fingers in the first example, the observation quality of the capsule endoscope 2 does not deteriorate.

Since the retainers 24 retain both edges of the body of the capsule endoscope 2 in the state of sandwiching each edge by the pair of contact parts 24b and thereby a function of retaining the capsule endoscope 2 can be secured adequately in the first example, the capsule endoscope 2 does not jump out from the package due to shaking or dropping of the package 20 and it is possible to stably retain the capsule endoscope 2.

### (Second example useful for understanding the invention)

Next, a second example useful for understanding the invention will be explained. While the example of implementing, in the retainer, both of the function of retaining edge part of the body of the capsule endoscope and the function of preventing each end of the capsule endoscope 2 from being grasped is explained in the first example, a case of providing a grasp preventive part separately from the retainer will be explained in the second embodiment.

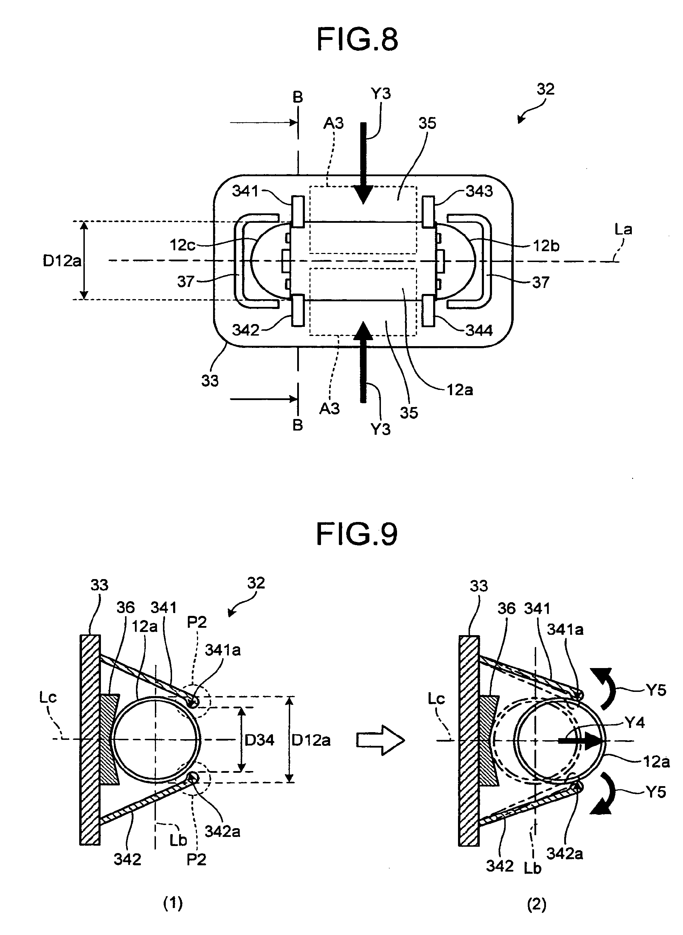

FIG. 7 is a perspective view of a base container that forms a package for a capsule endoscope according to the second example, FIG. 8 is a top view of an upper surface of the base container shown in FIG. 7, and FIG. 9 includes cross sectional views along the line BB in FIG. 8. A package for a capsule endoscope according to the second example has a configuration in which a main surface of a base container 32 shown in FIG. 7 is closed by the sterilization sheet 21 shown in FIG. 3 similarly to the first example.

As shown in FIGS. 7 to 9, retainers 341 to 344 are formed in the base container 32 in a manner of protruding upward from an upper surface of a base member 33 along a vertical axis of the upper surface of the base member 33. Among the retainers 341 to 344, the facing retainers 341 and 342 are provided in pairs at a left edge part of the body of the capsule endoscope 2 and the facing retainers 343 and 344 are provided in pairs at a right edge part of the body of the capsule endoscope 2. The retainers 341 to 344 retain both edges of the body of the capsule endoscope 2 in such a manner that the capsule endoscope 2 can be taken out from the base container 33 in a diametrical direction of the capsule endoscope 2 (in a direction along the axis Lc perpendicular to the upper surface of the base member 33 shown in FIG. 9). Each of the retainers 341 to 344 has a structure of protruding from the upper surface of the base member 33 in the direction along which the capsule endoscope 2 is taken out.

The retainers 341 and 342, as shown in an area P1 in FIG. 9, includes protruding parts 341a and 342a at end parts, respectively. The pair of protruding parts 341a and 342a locates above the body of the capsule endoscope 2 at the left edge of the body of the capsule endoscope 2. A distance D34 along the surface Lb between a distal end of the protrusion of the protruding part 341a and a distal end of the protrusion of the protruding part 342a facing the protruding part 341a is set to be smaller than the outer diameter of the body of the capsule endoscope 2 (the outer diameter D12a of the cylindrical casing 12a). Therefore, the pair of protruding parts 341a and 342a serves as a stopper which prevents a separation of the capsule endoscope 2 in the direction along which the capsule endoscope 2 is taken out. Besides, protruding parts are respectively provided in the retainers 343 and 344 similarly to the retainers 341 and 342.

In the state where the capsule endoscope 2 is arranged in the base container 32, the retainers 341 to 344 are not in contact with the capsule endoscope 2 and formed in a manner of retaining the capsule endoscope 2 in the base container 32 with an adequate force so as to allow taking out of the capsule endoscope 2 by a grasp with fingers from the base container 33.

As shown in FIG. 9, a position regulator 36 which is in contact with at least the edge part of the body of the capsule endoscope 2 is provided on the upper surface of the base member 33. The position regulator 36 is provided in a manner of being in contact with the cylindrical casing 12a of the capsule endoscope 2 along the long axis La. The position regulator 36 has a depression formed on a surface which is in contact with the capsule endoscope 2 such that a V-shaped cross section is formed as shown in FIG. 9. Since the capsule endoscope 2 is positioned at a center part of the depression when the capsule endoscope 2 is arranged on the position regulator 36, the position regulator 36 has a function of regulating the position of the capsule endoscope 2 not to move along the surface Lb. The position regulator 36 is provided not only at the left edge of the body of the capsule endoscope 2 but also at the right edge of the body of the capsule endoscope 2 correspondingly.

In the base container 32, two grasp preventive parts 37 are provided separately from the retainers 341 to 344. The grasp preventive parts 37 are formed to cover respective ends of the capsule endoscope 2 in the long axis La. Specifically, the grasp preventive parts 37 are formed around the dome-shaped casings 12b and 12c of the capsule endoscope 2 when seen from the top as shown in FIG. 8. As shown in FIG. 7, the grasp preventive parts 37 have a height almost equivalent to the outer diameter of the capsule endoscope 2 on the base member 33. The grasp preventive parts 37, by being formed in this manner, prevent both ends of the capsule endoscope 2 in the long axis from being grasped from the direction along the long axis La of the capsule endoscope 2. Since a movement of the capsule endoscope 2 in the direction along the long axis La is also regulated by the grasp preventive parts 37, the grasp preventive parts 37 also have a function of regulating a positional movement of the capsule endoscope 2 in the direction along the long axis La.

As shown in FIGS. 7 and 8, the retainers 341 and 343 as well as the retainers 342 and 344 are formed away from each other in a manner of allowing the body of the capsule endoscope 2 to be exposed. An area between the retainers 341 and 343 and an area between the retainers 342 and 344 serve as a non-retaining part 35. In an area A3 in the non-retaining part 35 in FIG. 8, the body of the capsule endoscope 2 is exposed allowing the grasp. When seen from the top as shown in FIG. 8, since the capsule endoscope 2 is covered by the retainers 341 to 344 and the grasp preventive parts 37 except for the non-retaining part 35, fingers of the operator are guided to the body of the capsule endoscope 2 exposed in the non-retaining part 35.

The operator who takes out the capsule endoscope 2, after the fingers are guided to the body of the capsule endoscope 2 exposed in the non-retaining part 35, grasps the body of the capsule endoscope 2 by having fingers approach like an arrow Y3 in FIG. 8. Then, the operator takes out the capsule endoscope 2 from the base container 32 along the axis Lc like an arrow Y4 shown in FIG. 9(2). In other words, when the base container 32 is seen from the top as shown in FIG. 8, the operator takes out the capsule endoscope 2 by raising it upward in a nearly vertical direction to the paper plane after having fingers approach like the arrow Y3 and grasping the body of the capsule endoscope 2 with the fingers.

In this case, a side surface of the body of the capsule endoscope 2 is in line contact or a point contact with the protruding parts 341a and 342a, the capsule endoscope 2 having moved along the axis Lc like the arrow Y4 shown in FIG. 9(2) due to the operation of taking out the capsule endoscope 2 by the operator. Thus, a force from an inner side to an outer side like an arrow Y5 from the side surface of the body of the capsule endoscope 2 is applied to the retainers 341 and 342 as shown in FIG. 9(2). Since the retainers 341 and 342 temporarily change in shape in a manner of being pressed and extended due to the force and the distance between the retainers 341a and 342a provided in pairs becomes long enough to be equivalent to or more than the outer diameter of the capsule endoscope 2, the capsule endoscope 2 can be taken out from the base container 32. The retainers 343 and 344 also change in shape in the same manner.

In this manner, since the function of retaining the capsule endoscope 2 is secured adequately and the capsule endoscope 2 is retained in the state where the body of the capsule endoscope 2 is widely exposed, the capsule endoscope 2 can be easily taken out, and since the grasp preventive parts 37 are formed around the dome-shaped casing 12b and 12c, the dome part does not get dirty in the second example similarly to the first example.

Furthermore, since the retainers 341 to 344 are not in contact with the capsule endoscope 2 in the state where the capsule endoscope 2 is arranged in the base container 32 in the second example, an area in which the capsule endoscope 2 and the base container 32 are in contact is smaller than the area in the first example. Therefore, the second example can further enhance a certainty of the sterilization since sterilizing gas is able to be in contact with the side surface of the capsule endoscope 2 adequately.

### (First modification of the second example)

Next, a first modification of the second example will be explained. In the first modification of the second example, a case of taking out the capsule endoscope not upward along the vertical axis of the upper surface of the base part but by sliding in parallel with the upper surface of the base part will be explained.

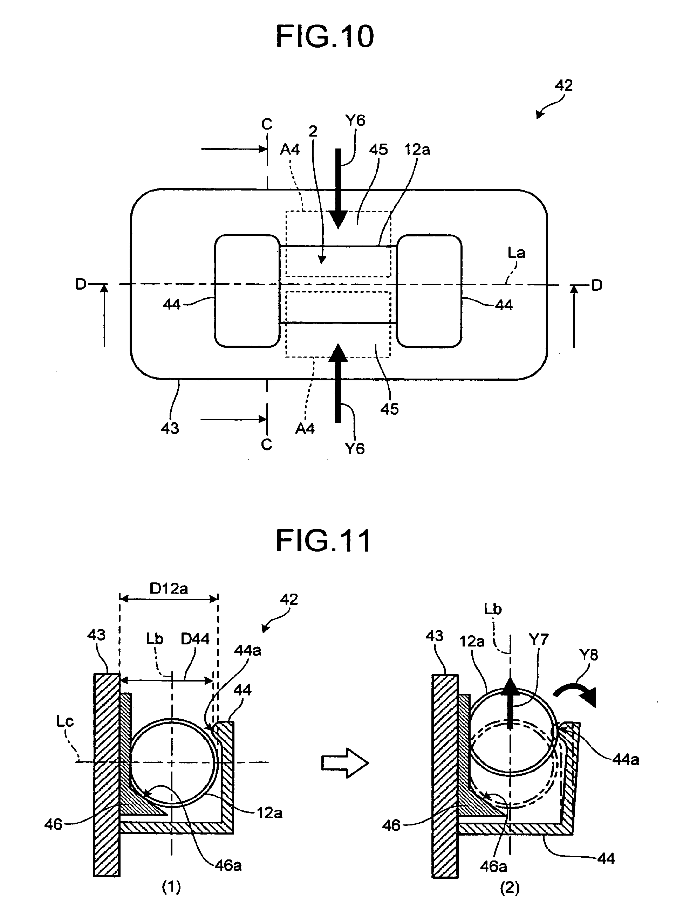

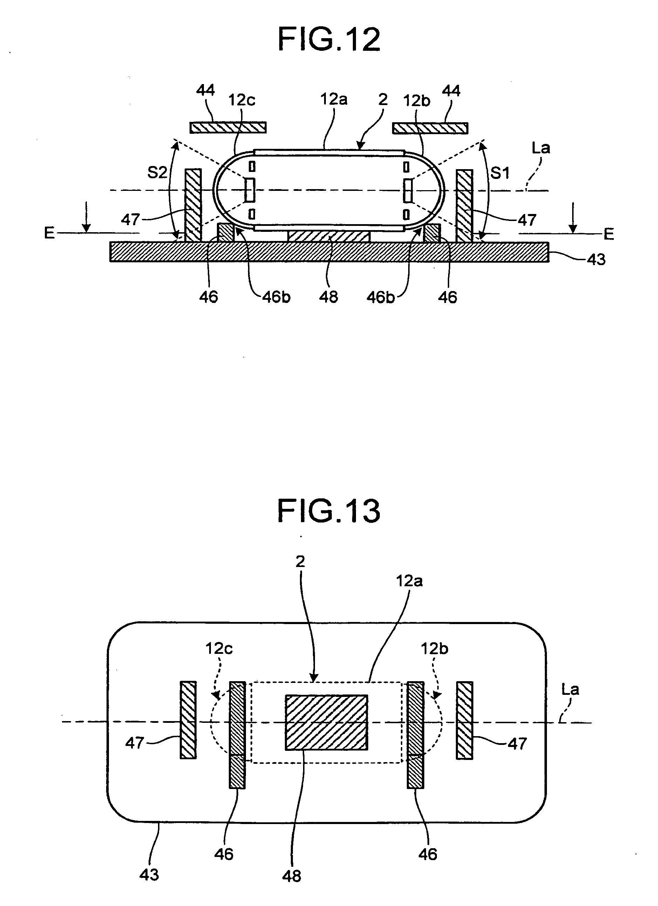

FIG. 10 is a perspective view of a base container that forms a package for a capsule endoscope according to the first modification of the second example, FIG. 11 includes cross sectional views along the line CC in FIG. 10, FIG. 12 is a cross sectional view along the line DD in FIG. 10, and FIG. 13 is a cross sectional view along the line EE in FIG. 12. A package for a capsule endoscope according to the first modification of the second example has a configuration in which a main surface of a base container 42 shown in FIG. 10 is closed by the sterilization sheet 21 shown in FIG. 3 similarly to the first example.

As shown in FIGS. 10 to 11, two retainers 44 each having an L-shape cross section are formed on an upper surface of a base member 43 in the base container 42. The two retainers 44 are provided at both edges of the body of the capsule endoscope 2, i.e., at both edges of the cylindrical casing 12a to retain both edges of the body of the capsule endoscope 2.

Each of the retainers 44 has a shape with an opening in a part at an upper side in the diametrical direction of the capsule endoscope 2 of the capsule endoscope 2 along the surface Lb as shown in FIG. 11. The retainers 44 retain the body of the capsule endoscope 2 in a manner of allowing the capsule endoscope 2 to be taken out in a direction to the opening part. Each of the retainers 44 has a shape also with an opening at both sides through which the long axis La shown in FIG. 10 goes.

Each of the retainers 44 has a protruding part 44a as shown in FIG. 11. The protruding part 44a of the retainer 44 locates above the body of the capsule endoscope 2 at the edge part of the body of the capsule endoscope 2. A distance D44 along the axis Lc between a distal end of the protrusion of the protruding part 44a and the upper surface of the base member 43 facing the protruding part 44a is set to be smaller than the outer diameter D12a of the body of the capsule endoscope 2. Therefore, the protruding part 44a serves as a stopper which prevents a separation of the capsule endoscope 2 in the direction along which the capsule endoscope 2 is taken out (in an upper direction along the surface Lb shown by an arrow Y7 in FIG. 11(2)).

In the state where the capsule endoscope 2 is arranged in the base container 42, the retainers 44 are not in contact with the capsule endoscope 2 and formed in a manner of retaining the capsule endoscope 2 in the base container 42 with an adequate force so as to allow taking out of the capsule endoscope 2 by a grasp with fingers from the base container 42.

As shown in FIGS. 11 to 13, a position regulator 46 which has an L-shape cross section is provided on the upper surface of the base member 43. The position regulator 46 is in contact with an area other than the imaging fields S1 and S2 in the outer surfaces of the dome-shaped casings 12b and 12c of the capsule endoscope 2. The position regulator 46 is formed in such a manner that a surface 46a which has contact with the dome part of the capsule endoscope 2 is moderately inclined as shown in FIG. 11. Thus, the position of the capsule endoscope 2 is regulated not to move along the surface Lb. When the capsule endoscope 2 is arranged on the position regulator 46, positioning is made in such a manner that the dome-shaped casings 12b and 12c of the capsule endoscope 2 get stuck with a corner 46b of the surface 46a. Therefore, the position regulator 46 regulates the position of the capsule endoscope 2 not to move in the direction along the long axis La as well as regulating the position of the capsule endoscope 2 not to move along the surface Lb.

As shown in FIGS. 12 and 13, two grasp preventive parts 47 are provided separately from the retainers 44 on the upper surface of the base member 43. As shown in FIGS. 12 and 13, the grasp preventive parts 47 are formed at the outer sides of respective ends of the capsule endoscope 2 in the long axis La and prevent the dome-shaped casings 12b and 12c from being grasped in the direction along the long axis La. Therefore, the grasp preventive parts 47 prevent both ends of the capsule endoscope 2 in the long axis from being grasped from the direction along the long axis La of the capsule endoscope 2. Since the movement of the capsule endoscope 2 in the direction along the long axis La is regulated by the grasp preventive parts 47, the grasp preventive parts 47 also have a function of regulating the positional movement of the capsule endoscope 2 to the direction along the long axis La. A supporting plate 48 on which the body of the capsule endoscope 2 locates is provided on the upper surface of the base member 43.

Besides, the retainers 44 are formed away from each other in a manner of allowing the body of the capsule endoscope 2 to be exposed, similarly to the first embodiment. An area not occupied by the retainer 44 between the retainers 44 serves as a non-retaining part 45. In an area A4 in the non-retaining part 45 in FIG. 10, the body of the capsule endoscope 2 is exposed allowing the grasp. As shown in FIGS. 10 to 13, since the capsule endoscope 2 is covered by the retainers 44 and the grasp preventive parts 47 except for the non-retaining part 45, fingers of the operator are guided to the body of the capsule endoscope 2 exposed in the non-retaining part 45.

The operator who takes out the capsule endoscope 2, after the fingers are guided to the body of the capsule endoscope 2 exposed in the non-retaining part 45, grasps the body of the capsule endoscope 2 by having fingers approach like an arrow Y6 in FIG. 10. Then, the operator takes out the capsule endoscope 2 from the base container 42 upward along the surface Lb like an arrow Y7 shown in FIG. 11(2). In other words, when the base container 42 is seen from the top as shown in FIG. 10, the operator takes out the capsule endoscope 2 by sliding it upward in parallel with the paper plane after having fingers approach like the arrow Y6 and grasping the body of the capsule endoscope 2 with the fingers.

In this case, a side surface of the body of the capsule endoscope 2 is in line contact or a point contact with the protruding parts 44a, the capsule endoscope 2 having moved along the surface Lb like the arrow Y7 shown in FIG. 11(2) due to the operation of taking out the capsule endoscope 2 by the operator. Thus, a force from an inner side to an outer side like an arrow Y8 is applied from the side surface of the body of the capsule endoscope 2 to the retainers 44 as shown in FIG. 11(2). Since the retainers 44 temporarily change in shape in a manner of being pressed and extended due to the force and the distance between the protruding parts 44a and the upper surface of the base member 43 becomes long enough to be equivalent to or more than the outer diameter of the capsule endoscope 2, the capsule endoscope 2 can be taken out from the base container 42.

In this manner, since the function of retaining the capsule endoscope 2 is secured adequately and the capsule endoscope 2 is retained in the state where the body of the capsule endoscope 2 is widely exposed, the capsule endoscope 2 can be easily taken out, and since the grasp preventive parts 47 are formed around the dome-shaped casing 12b and 12c, the dome part does not get dirty in the first modification of the second example similarly to the second example. Since the retainers 44 are not in contact with the capsule endoscope 2 in the state where the capsule endoscope 2 is arranged in the base container 42 also in the first modification of the second example, an area in which the capsule endoscope 2 and the base container 42 are in contact is smaller than the area in the first embodiment and therefore it is possible to further enhance a certainty of the sterilization.

### (Third example useful for understanding the invention)

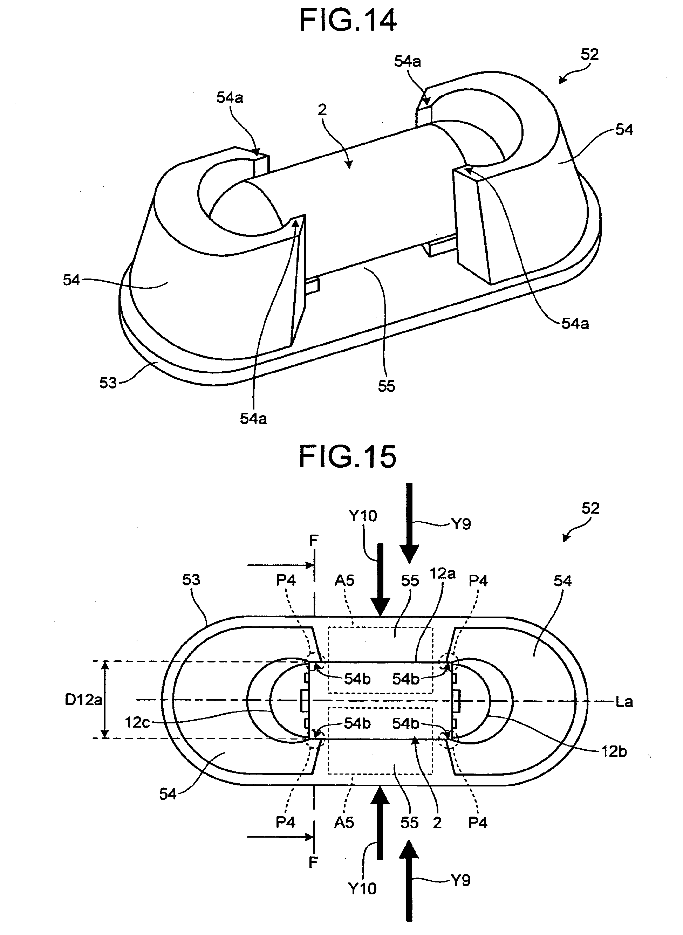

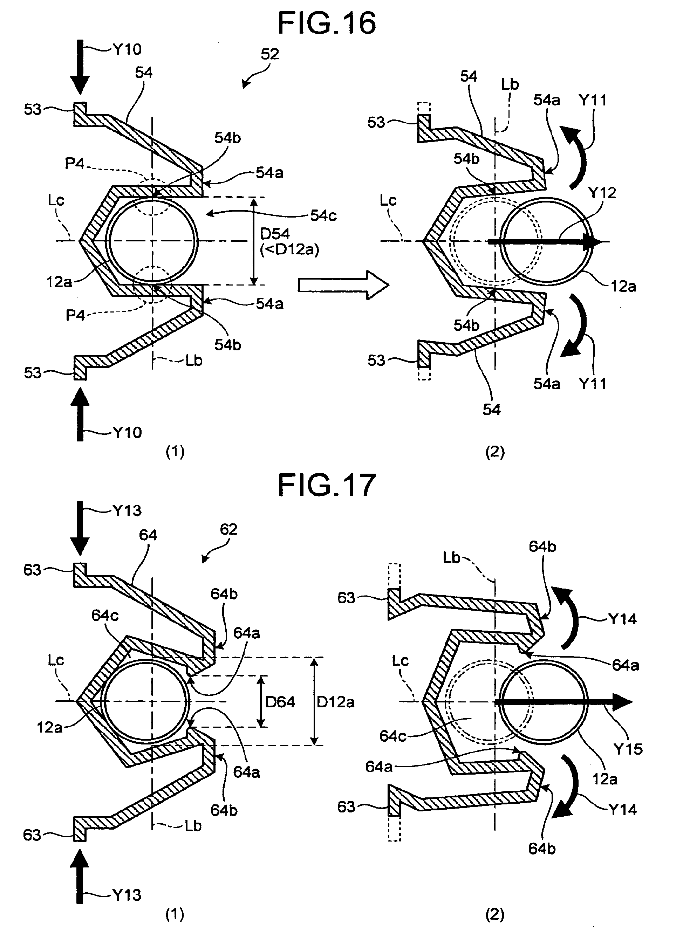

Next, a third example useful for understanding the invention will be explained. In the third embodiment, a case where a retainer and a base part are integrally formed by using a thin resin material and the like will be explained. FIG. 14 is a perspective view of a base container that forms a package for a capsule endoscope according to the third example, FIG. 15 is a top view of an upper surface of the base container shown in FIG. 14, and FIG. 16 includes cross sectional views along the line FF in FIG. 15. A package for a capsule endoscope according to the third example has a configuration in which a main surface of a base container 52 shown in FIG. 14 is closed by the sterilization sheet 21 shown in FIG. 3 similarly to the first example.

As shown in FIGS. 14 to 16, two retainers 54 are formed in the base container 52 in a manner of protruding upward from an upper surface of a base member 53 along a vertical axis of the upper surface of the base member 53. The two retainers 54 are provided at both edges of the body of the capsule endoscope 2 to retain both edges of the body of the capsule endoscope 2. The two retainers 54 retain the body of the capsule endoscope 2 in such a manner that the capsule endoscope 2 can be taken out from the base container 52 in a diametrical direction of the capsule endoscope 2 (in a direction along the axis Lc shown in FIG. 16). Each of the retainers 54 has a structure of protruding from the upper surface of the base member 53 in the direction along which the capsule endoscope 2 is taken out.

The retainers 54 are formed to cover respective ends of the capsule endoscope 2 in the long axis La. As shown in FIG. 15, the retainers 54 are formed around the dome-shaped casings 12b and 12c of the capsule endoscope 2 when seen in the top view. As shown in FIG. 16, the retainers 54 have a height almost equivalent to the outer diameter of the capsule endoscope 2 on the base member 53. Thus, the retainers 54 prevent both ends of the capsule endoscope 2 in the long axis from being grasped from the direction along the long axis La of the capsule endoscope 2.

As shown in FIGS. 15 and 16, each of the retainers 54 has a pair of contact parts 54b which are in contact with a part of an edge part of the body of the capsule endoscope 2. The pair of contact parts 54b of the retainer 54 is configured to mutually put the body of the capsule endoscope 2 therebetween. The retainer 54 sandwiches the edge part of the body of the capsule endoscope 2 by two facing surfaces on which respective contact parts 54b locate. In the example shown in FIG. 16, the pair of contact parts 54b and the edge part of the body of the capsule endoscope 2 are in line contact or in a point contact in a cross line between the surface Lb and an inner wall of the retainer 54 as shown in an area P4.

The retainer 54 is formed in such a manner that a distance D54 (see FIG. 16) along the surface Lb between the contact parts 54b provided in pairs is smaller than the outer diameter D12a (see FIG. 15) of the cylindrical casing 12a of the capsule endoscope 2. Therefore, the pair of contact parts 54b supports the capsule endoscope 2 in a retaining area 54c between the retainers 54 by a friction generated with the body of the capsule endoscope 2 so that the capsule endoscope 2 does not involuntarily jump out in the direction along which the capsule endoscope 2 is taken out (the direction along the axis Lc shown in FIG. 16).
The retainer 54 is formed to support the capsule endoscope 2 with an adequate force so as to allow taking out of the capsule endoscope 2 by a grasp with fingers from the base container 52.

The retainers 54 are formed away from each other in a manner of allowing the body of the capsule endoscope 2 to be exposed. An area not occupied by the retainer 54 between the retainers 54 serves as a non-retaining part 55. In an area A5 in the non-retaining part 55 in FIG. 15, the body of the capsule endoscope 2 is exposed allowing the grasp. When seen from the top as shown in FIG. 15, since the capsule endoscope 2 is covered by the retainers 54 except for the non-retaining part 55, fingers of the operator are guided to the body of the capsule endoscope 2 exposed in the non-retaining part 55.

Furthermore, the base member 53 and the retainers 54 are formed integrally as shown in FIG. 16. The base member 53 and the retainers 54 are formed by a thin resin material such as polypropylene, for example. Therefore, when the base member 53 is depressed from both sides along the surface Lb like an arrow Y10 shown in FIGS. 15 and 16, the base member 53 is pressed to the inner side and changes in shape and accordingly the retainers 54 also change in shape in such a manner that upper surfaces 54a of the retainers 54 stretch out as shown by an arrow Y11 in FIG. 16(2).

The operator who takes out the capsule endoscope 2, after the fingers are guided to the body of the capsule endoscope 2 exposed in the non-retaining part 55, grasps the body of the capsule endoscope 2 by having fingers approach like an arrow Y9 in the area A5 in the non-retaining part 55. Then, the operator depresses the base member 53 from both sides along the surface Lb like the arrow Y10 in FIGS. 15 and 16. As a result of this, the base member 53 is pressed toward the inner side and changes in shape and accordingly the retainers 54 also change in shape in such a manner that the upper surfaces 54a of the retainers 54 temporarily stretch out. This change in shape enables the distance between the contact parts 54b provided in pairs to become long enough to be equivalent to or more than the outer diameter of the capsule endoscope 2 like the arrow Y11 in FIG. 16(2) and the capsule endoscope 2 to be taken out from the base container 52 like an arrow Y12 (see FIG. 16).

In this manner, since the function of retaining the capsule endoscope 2 is secured adequately and the capsule endoscope 2 is retained in the state where the body of the capsule endoscope 2 is widely exposed, the capsule endoscope 2 can be easily taken out, and since the retainers 54 are formed around the dome-shaped casing 12b and 12c, the dome part does not get dirty also in the third example similarly to the first example.

Furthermore, since the capsule endoscope 2 is taken out by widening the distance between the retainers 54 via the depression of the base member 53 and therefore a small force to be applied to the capsule endoscope 2 will do for taking out the capsule endoscope 2 in the third example compared to the first and the second example, it is possible to further enhance an ease in taking out the capsule endoscope 2.

While the distance between the retainers 54 is configured to be deformed and widened by depressing the base member 53 in the third example, a mechanism allowing the retainers 54 to be automatically opened and closed may be provided and the capsule endoscope 2 may be taken out when the retainers 54 are opened.

Besides, a protruding part which functions as a stopper which prevents the capsule endoscope 2 from jumping out in the direction along which the capsule endoscope 2 is taken out may be provided, in addition to putting the capsule endoscope 2 between the contact parts 54b provided in pairs to retain the capsule endoscope 2 in the third embodiment. FIG. 17 includes cross sectional views of another example of the base container according to the third example and show a cross section of another example of the base container along the same line as the line FF in FIG. 15.

As shown in FIG. 17, two retainers 64 which are integrally formed with a base member 63 are provided in a base container 62 similarly to the base container 52 and each of the retainers 64 is provided with a pair of protruding parts 64a. The retainer 64 is formed in such a manner that a distance D64 along the surface Lb between protruding tip ends of the protruding parts 64a becomes smaller than the outer diameter D12a of the body of the capsule endoscope 2. Since the base member 63 and the retainers 64 are integrally formed by a thin resin material similarly to the third example, when the base member 63 is depressed from both sides along the surface Lb like an arrow Y13 in FIG. 17(1), the base member 63 is pressed toward the inner side and changes in shape and accordingly the retainers 64 also change in shape in such a manner that upper surfaces 64b of the retainers 64 stretch out. In response to this, the distance between the protruding parts 64a of the retainer 64 becomes long enough to be equivalent to or more than the outer diameter of the capsule endoscope 2 like an arrow Y14 in FIG. 17(2) and the capsule endoscope 2 in a retaining area 64c can be taken out from the base container 62 like an arrow Y15.

### (Fourth example and embodiment of the invention)

Next, a fourth example and embodiment of the invention will be explained. A case of taking out the capsule endoscope not in the diametrical direction but in the longitudinal direction of the capsule endoscope will be explained in the fourth example and embodiment of the invention.

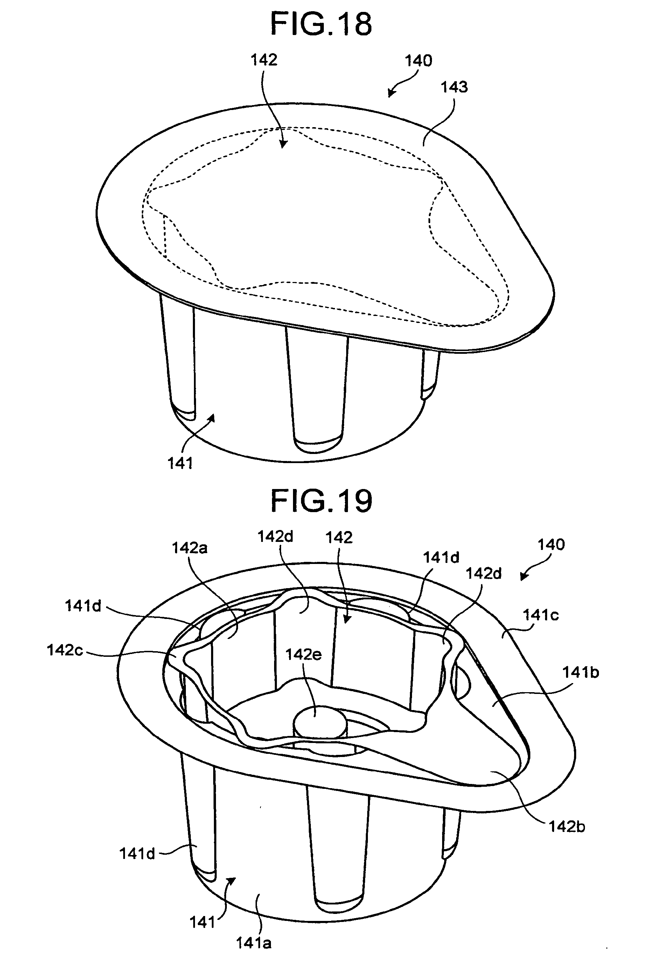

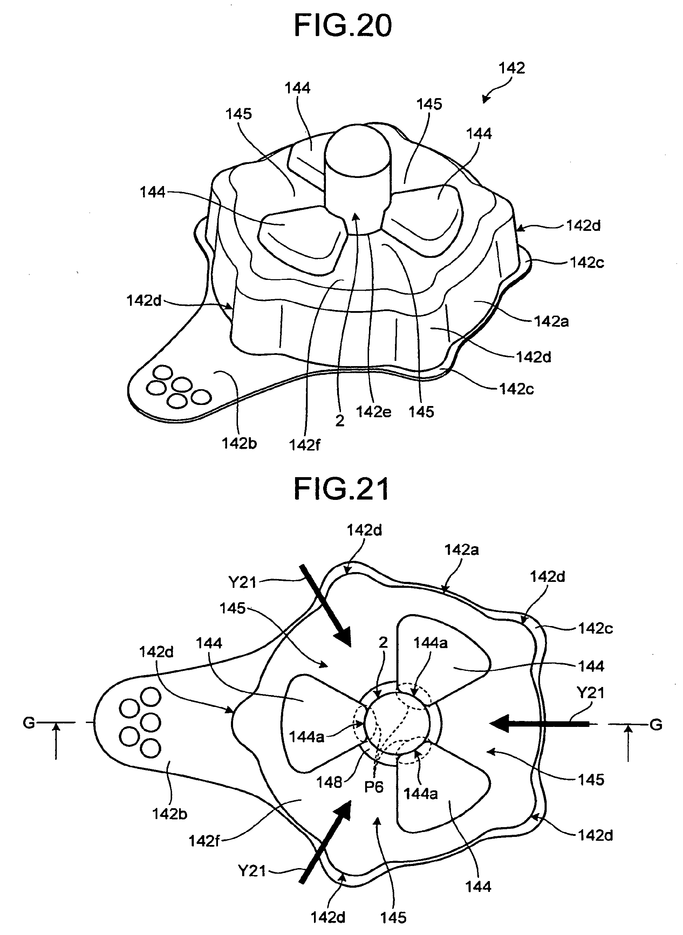

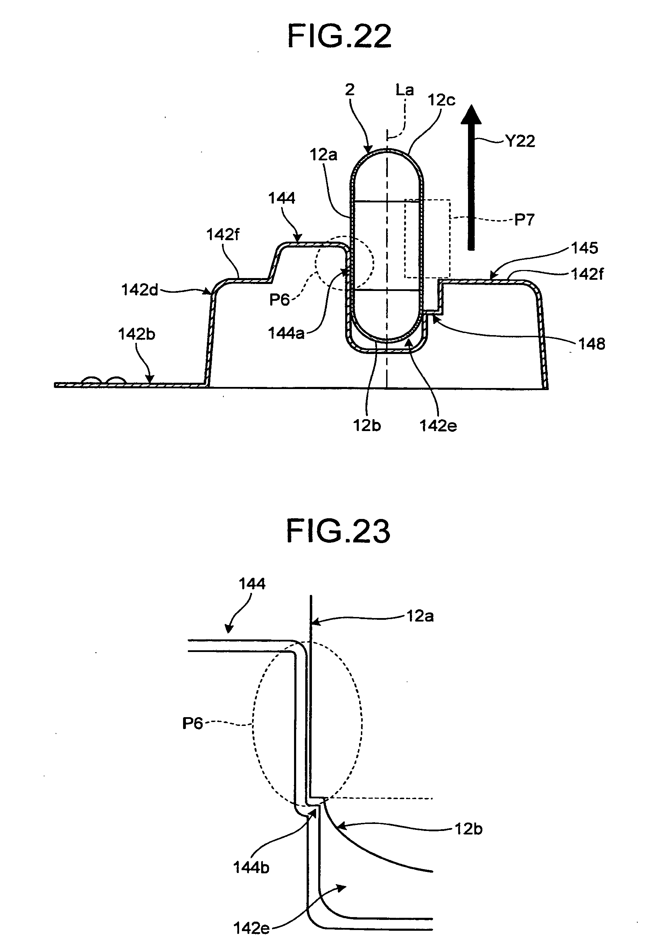

FIG. 18 is a perspective view of a configuration of a package for a capsule endoscope according to the fourth example and embodiment of the invention, FIG. 19 is a perspective view of one example of a case where the sterilization sheet is removed from the package shown in FIG. 18, FIG. 20 is a perspective view of the base container shown in FIG. 19 and shows a case where the base container in the state shown in FIG. 19 is turned upside down, FIG. 21 is a top view of an upper ! surface of the base container shown in FIG. 20, and FIG. 22 is a cross sectional view along the line GG in FIG. 21.

As shown in FIGS. 18 and 19, a package 140 is provided with a blister pack 141 as an outer housing part, a base container 142 which is fitted in the blister pack 141 and serves as an inner housing part that retains the capsule endoscope 2, and a sterilization sheet 143 which is provided over an upper surface of the blister pack 141 and closes an opening of the blister pack 141.

The blister pack 141 includes a cylindrical part 141a having a bottom, a handgrip part 141b which has a tongue like shape and is provided on a part of an upper edge of an opening of the cylindrical part 141a, an edge part 141c provided along an outer circumference of the upper edge of the opening of the cylindrical part 141a and the handgrip part 141b, and a plurality of protruding parts 141d which are provided on an outer circumferential surface of the cylindrical part 141a and each have nearly semi-cylindrical shape formed along a long side direction of the cylindrical part 141a in a manner of protruding from an inside to an outside of the cylindrical part 141a. The handgrip part 141b is formed of a plate member having an approximately triangular upper surface and configured so that a handgrip part 142b of the base container 142 to be described later can abut thereon. The edge part 141c has a predetermined width and is formed in a step shape higher by one step along the outer circumference of the upper edge of the opening of the cylindrical part 141a and the handgrip part 141b. A height of the edge part 141c is configured to be equivalent to or more than a thickness of the handgrip part 142b and an edge part 142c of the base container 142 abutting on the handgrip part 141b and the sterilizing sheet 143 is attached to the upper surface of the edge part 141c in a state where the base container 142 is housed in the blister pack 141.

Next, the base container 142 will be explained with reference to FIGS. 20 to 22. As shown in FIGS. 20 to 22, the base container 142 includes a cylindrical part 142a having a bottom, the handgrip part 142b which has a tongue like shape and is provided on a part of an upper edge of an opening of the cylindrical part 142a, the edge part 142c provided in a manner of extending from the handgrip part 142b on the upper edge of the opening of the cylindrical part 142a, and a plurality of protruding parts 142d which each have nearly semi-cylindrical shape protruding from an inside toward an outside of the cylindrical part 142a. A base surface 142f as a main surface is formed in the cylindrical part 142a and a hole 142e which has a bottom and into which one end of the capsule endoscope 2 in the long axis La is fitted and mounted is provided at a center of the base surface 142f. As shown in FIG. 22, a height of the base surface 142f is set to be almost equivalent to the height of the edge part of the body of the capsule
endoscope 2 fitted and mounted into the hole 142e. An inner diameter of the hole 142e is slightly larger than an outer diameter of the capsule endoscope 2 so that the capsule endoscope 2 can be fitted and mounted therein.

In addition, a plurality of convex parts protruding from the base surface 142f uninterruptedly with a part of a side wall of the hole 142e are formed as retainers 144 on the base surface 142f. The retainers 144 are formed in a manner of protruding up to the vicinity of the center of the body of the capsule endoscope 2 fitted and mounted into the hole 142e. In the fourth example and embodiment of the invention, a case where three retainers 144 are formed will be taken as an example and explained.

Each of the retainers 144 has a fan like shape of stretching out towards an outer circumference of the base container 142. The retainers 144 are in contact with the edge part of the body of the capsule endoscope 2 fitted and mounted into the hole 142e in respective contact parts 144a which are uninterrupted from the hole 142e as shown in an area P6 in FIGS. 21 and 22. Each of the contact parts 144a of the three retainers 144 are in contact with the side surface of the edge part of the body of the capsule endoscope 2. Therefore, the edge part at one side of the body of the capsule endoscope 2 is retained at three spots by the three retainers 144 in the base container 142. The retainers 144 retain the one edge part of the body of the capsule endoscope 2 in a manner of allowing the capsule endoscope 2 to be taken out from the base container 142 in the longitudinal direction of the capsule endoscope 2 (in the direction along the long axis La of the capsule endoscope 2 shown in FIG. 22). Thus, each of the retainers 144 has a structure of protruding from the base surface 142f in the direction along which the capsule endoscope 2 is taken out.

The retainers 144 are formed away from each other in a manner of allowing the body of the capsule endoscope 2 to be exposed and an area not occupied by the retainer 144 among the retainers 144 serves as a non-retaining part 145. In the non-retaining part 145 as shown in an area P7 in FIG. 22, the body of the capsule endoscope 2 is exposed allowing the grasp.

Besides, when the base member 144 is seen from the top as shown in FIG. 21, since each of the retainers 144 has the fan like shape, which has a narrower width from the outer circumference of the base container 142 toward the side surface of the capsule endoscope 2, the non-retaining part 145 has a shape, which has a narrower width from the outer circumference of the base container 142 toward the side surface of the capsule endoscope 2. Therefore, when fingers of the operator approach the side surface of the capsule endoscope 2 like an arrow Y21 in FIG. 21, three fingers are guided, in a manner of grasping the outer circumferential part of the capsule endoscope 2 at three spots separated by 120 degrees, along the shape of the retainers 144 to the side surface of the body of the capsule endoscope 2 exposed in the non-retaining part 145.

The operator, after the three fingers are guided to the side surface of the body of the capsule endoscope 2, grasps the body of the capsule endoscope 2 directly at the three spots to raise and take out the capsule endoscope 2 along the long axis La of the capsule endoscope 2 like an arrow Y22 in FIG. 22 from the base container 142. As shown in FIGS. 21 and 22, a step 148 is formed in an area other than the area where the retainers 144 are formed around the hole 142e to make it easy to grasp and take out the capsule endoscope 2.

Since the bottom part of the hole 142e covers the one end of the capsule endoscope 2 in the long axis La when the capsule endoscope 2 is fitted and mounted into the hole 142e, the bottom part of the hole 142e also has a function of preventing the one end of the capsule endoscope 2 in the long axis from being grasped from the direction along the long axis La of the capsule endoscope 2.

In this manner, the retainers 144 retain the capsule endoscope 2 in the state of allowing the body of the capsule endoscope 2 to be widely exposed in the direction along which the fingers approach and in the depth direction in the fourth example and embodiment of the invention. Therefore, since fingers can easily approach the side surface of the body and grasp the side surface of the body as far as a lower side of the body in the fourth example and embodiment of the invention, the capsule endoscope 2 can be easily taken out by fingers from the base container 142.

In the fourth example and embodiment of the invention, since one of the dome-shaped casings 12b and 12c is covered by the bottom part of the hole 142e when the capsule endoscope 2 is fitted and mounted into the hole 142e, the fingers do not have contact with the one of the dome-shaped casings 12b and 12c and the dome part does not get dirty. Besides, the fingers of the operator are guided to the side surface of the body of the capsule endoscope 2 along the shape of the retainers 144 in the fourth example and embodiment of the invention. Here, since the other one of the dome-shaped casings 12b and 12c not fitted and mounted into the hole 142e does not locate in a pathway of the guidance, there are few possibilities that the fingers of the operator have contact with it and the dome part not fitted and mounted into the hole 142e gets dirty.

In the fourth example and embodiment of the invention, three retainers 144 are in contact with the edge part of the body of the capsule endoscope 2. Thus, since the edge part of the body of the capsule endoscope 2 is retained at three spots, the function of retaining the capsule endoscope 2 can be secured adequately in the fourth example and embodiment of the invention.

In the fourth example and embodiment of the invention, it is possible to restrict a positional movement in the depth direction of the capsule endoscope 2 by providing a step 144b on an uninterrupted surface, with the hole 142e, of the retainers 144 and causing a joint part between the cylindrical casing 12a and the dome-shaped casing 12b to get stuck in the step 144b as shown in FIG. 23. This allows preventing a top part of the dome-shaped casing 12b from being in contact with the bottom surface of the hole 142e. When there is no such joint part in the capsule endoscope 2, the positional movement in the depth direction of the capsule endoscope 2 may be restricted similarly as described above by causing a position which is out of the range of the angle of field of the imaging unit in the dome-shaped casing 12b (a position which is not captured in an image) to be in direct contact with the step 144b.

The fourth example and embodiment of the invention is explained by taking as an example the base container 142 in which the retainers 144 each having the fan like shape are formed when seen from the top as shown in FIG. 21.

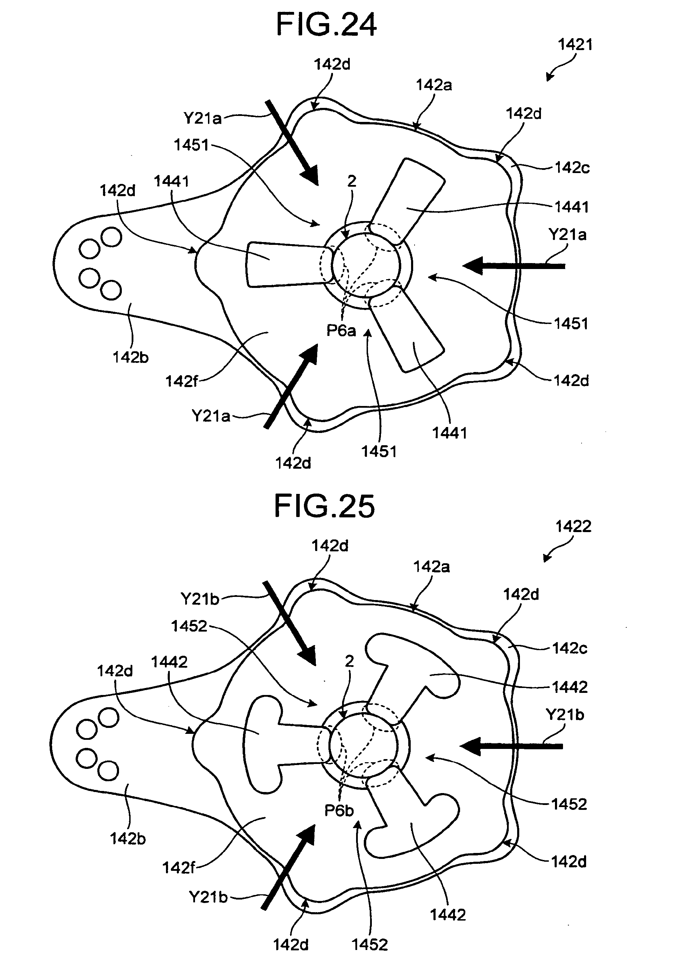

For example, a retainer 1441 having an I shape may be adopted as shown in a base container 1421 in FIG. 24. Since the edge part of the body of the capsule endoscope 2 is retained at three spots by three retainers 1441 in an area P6a in this case, the function of retaining the capsule endoscope 2 can be secured adequately. The retainers 1441 are formed away from each other in a manner of allowing the body of the capsule endoscope 2 to be exposed and the fingers of the operator are guided from the outer side of the capsule endoscope 2 to the side surface of the body of the capsule endoscope 2 in a non-retaining part 1451 not occupied by the retainer 1441 among the retainers 1441 as shown by an arrow Y21a.

A retainer 1442 having a T shape may be adopted as shown in a base container 1422 in FIG. 25. Since the edge part of the body of the capsule endoscope 2 is retained at three spots by three retainers 1442 in an area P6b in this case, the function of retaining the capsule endoscope 2 can be secured adequately. The retainers 1442 are formed away from each other in a manner of allowing the body of the capsule endoscope 2 to be exposed and the fingers of the operator are guided from the outer side of the capsule endoscope 2 toward the side surface of the body of the capsule endoscope 2 in a non-retaining part 1452 not occupied by the retainer 1442 among the retainers 1442 as shown by an arrow Y21b. While the case of providing three retainers as retainers 144, retainers 1441, and retainers 1442 is taken as an example and explained in the fourth example and embodiment of the invention, the present invention is not, of course, limited to this configuration and it is only necessary to provide a plurality of retainers so that the capsule endoscope 2 can be retained.

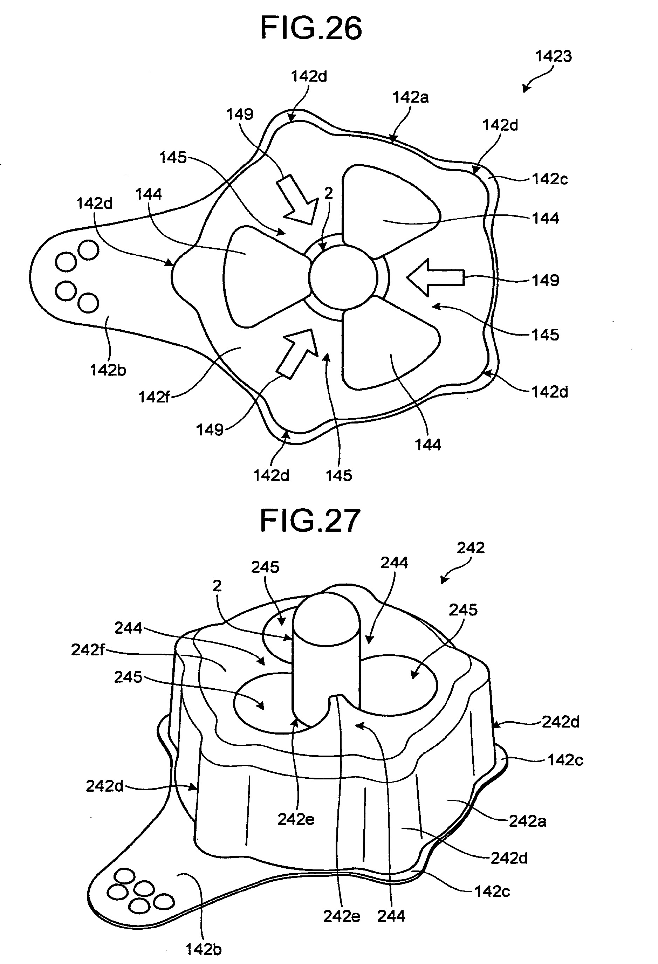

As shown in a base container 1423 in FIG 26, an arrow mark 149 indicating a grasp direction may be shown in the non-retaining part 145 on the base surface 142f. In this case, the operator has the fingers approach, while visually checking the mark 149, in a direction indicated by the mark 149 and thereby can have the fingers approach the body of the capsule endoscope 2 accurately.

### (Fifth example useful for understanding the invention)

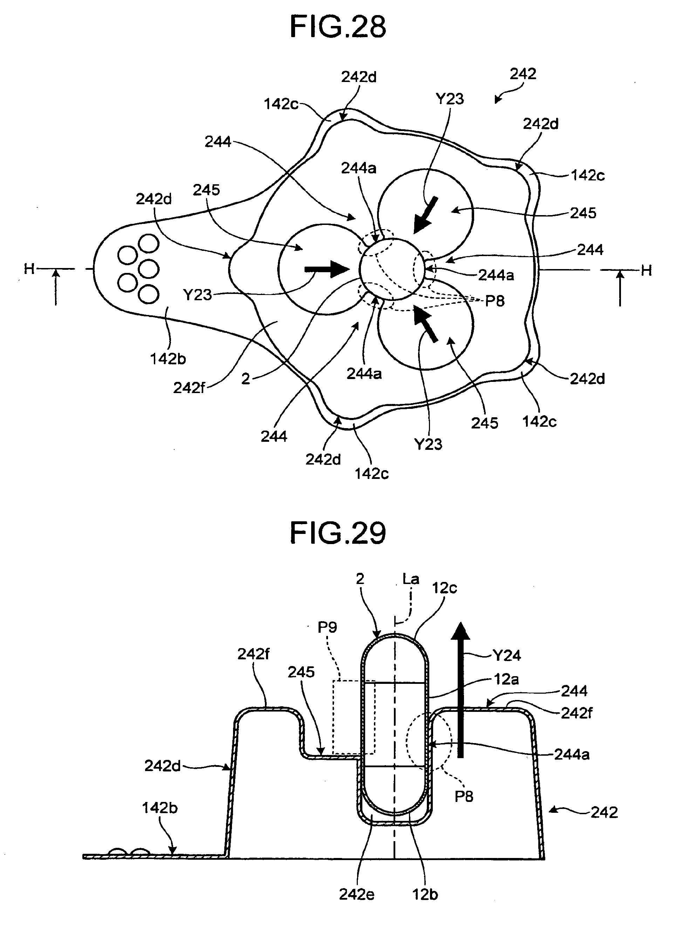

Next, a fifth example useful for understanding the invention will be explained. FIG. 27 is a perspective view of a base container that forms a package for a capsule endoscope according to the fifth example, FIG. 28 is a top view of an upper surface of the base container shown in FIG. 27, and FIG. 29 is a cross sectional view along the line HH in FIG. 28. A package for a capsule endoscope according to the fifth example has a configuration in which a base container 242 shown in FIG. 27 is mounted onto the blister pack 141 in a state of being turned upside down from the state shown in FIG. 27 and the opening of the blister pack 141 is closed by the sterilization sheet 143 similarly to the fourth example and embodiment of the invention.

As shown in FIG. 27, the base container 242 according to the fifth example includes, similarly to the base container 142 according to the fourth example and embodiment of the invention, the handgrip part 142b and the edge part 142c. The base container 242 includes a cylindrical part 242a having a bottom and a plurality of protruding parts 242d each of which has nearly semi-cylindrical shape formed in a manner of protruding from an inside toward an outside of the cylindrical part 242a.

A base surface 242f as a main surface is formed in the cylindrical part 242a and a hole 242e which has a bottom and into which one end of the capsule endoscope 2 in the long axis La and the body of the capsule endoscope 2 are fitted and mounted is provided at a center of the base surface 242f. As shown in FIG. 29, a height of the base surface 242f is set to be almost equivalent to the height of the middle part of the body of the capsule endoscope 2 fitted and mounted into the hole 242e. An inner diameter of the hole 242e is slightly larger than an outer diameter of the capsule endoscope 2 so that the capsule endoscope 2 can be fitted and mounted therein.

Besides, concave parts each of which has nearly circular shape are formed on the base surface 242f in such a manner that a part around the hole 242e on the base surface 242f is dug down uninterruptedly with a part of a side wall of the hole 242e. The concave part serves as a non-retaining part 245 and three concave parts are formed as the non-retaining parts 245 in the example shown in FIGS. 27 to 29. As shown in FIG. 29, the non-retaining part 245 is formed in a manner of being dug down to the edge part at a lower side of the body of the capsule endoscope 2 fitted and mounted into the hole 242e. Therefore, the body of the capsule endoscope 2 fitted and mounted into the hole 242e is exposed in an area corresponding to the non-retaining part 245 (area P9 shown in FIG. 29).

The non-retaining parts 245 are formed away from each other in a manner of being dug down while leaving a part of the side wall of the hole 242e and an area except for the concave parts (non-retaining parts 245) around the hole 242e on the base surface 242f serves as a retainer 244. In the retainer 244 as shown in an area P8 in FIGS. 28 and 29, a side wall 244a is in contact with a part of the side surface of the edge part of the body of the capsule endoscope 2 fitted and mounted into the hole 242e. Respective side walls 244a at three retainers 244 are in contact with a part of the side surface of the edge part of the body of the capsule endoscope 2. Therefore, one edge part of the body of the capsule endoscope 2 is retained at three spots by the three retainers 244 in the base container 242. The retainers 244 retain one edge part of the body of the capsule endoscope 2 in such a manner that the capsule endoscope 2 can be taken out from the base container 242 in the longitudinal direction of the capsule endoscope 2 (in the direction along the long axis La of the capsule endoscope 2 shown by an arrow Y24 in FIG. 29).

The fingers of the operator is inserted, from an upper direction of the base container 242, into the respective non-retaining parts 245 each opening of which is widely opened and grasps, after being guided to the side surface of the body of the capsule endoscope 2 like an arrow Y23, the body of the capsule endoscope 2 directly at the three spots to raise and take out the capsule endoscope 2 from the base container 242 along the long axis La of the capsule endoscope 2 like the arrow Y24 in FIG. 29.

In this manner, since the capsule endoscope 2 is retained in the base container 242 in a manner of allowing the body of the capsule endoscope 2 to be exposed adequately at three spots, it is possible in the fifth example to secure an area to be grasped by fingers and take out the capsule endoscope 2 from the base container 242 stably.

Since one of the dome-shaped casings 12b and 12c is covered by the bottom part of the hole 242e in the fifth example, similarly to the fourth example and embodiment of the invention, the fingers do not have contact with the one of the dome-shaped casings 12b and 12c and the dome part gets dirty.

Since the edge part of the body of the capsule endoscope 2 is retained at three spots by the three retainers 244 in the fifth example, similarly to the fourth embodiment, the function of retaining the capsule endoscope 2 can be secured adequately.

While the case of providing three concave parts to form three retainers 244 is taken as an example and explained in the fifth example, the present invention is not, of course, limited thereto and it is only necessary to provide a plurality of concave parts in a manner of allowing the capsule endoscope 2 to be retained.

While the case of housing the capsule endoscope 2 of binocular type is taken as an example and explained in the package for a capsule endoscope according to the first to the fifth example, the present invention is not, of course, limited to this configuration and a so-called monocular capsule endoscope may be housed.

### Reference Signs List

1 SUBJECT
2 CAPSULE ENDOSCOPE
3 RECEIVING DEVICE
3a RECEIVING JACKET
3b EXTERNAL DEVICE
4 DISPLAY DEVICE
5 PORTABLE RECORDING MEDIUM
11A, 11B IMAGING UNIT
12 CAPSULE CASING
12a CYLINDRICAL CASING
12b, 12c DOME-SHAPED CASING
13A, 13B ILLUMINATION UNIT
14A, 14B OPTICAL SYSTEM
15A, 15B IMAGING ELEMENT
16 RADIO COMMUNICATION UNIT
16a ANTENNA
17 CONTROL UNIT
18 POWER SOURCE UNIT
20, 140 PACKAGE
21, 143 STERILIZATION SHEET
22, 32, 42, 52, 62, 142, 242, 1421, 1422, 1423 BASE CONTAINER
23, 33, 43, 53 BASE MEMBER
24, 341-344, 44, 54, 64, 144, 244, 1441, 1442 RETAINER
24a INCLINATION SURFACE
24b, 54b, 144a CONTACT PART
25, 35, 45, 55, 145, 245, 1451, 1452 NON-RETAINING PART
26 SUPPORTING PLATE
36, 46 POSITION REGULATOR
37, 47 GRASP PREVENTIVE PART
44a, 64a, 141d, 142d, 242d, 341a, 342a PROTRUDING PART
46a SURFACE
54a, 64b UPPER SURFACE
63 BASE MEMBER
141 BLISTER PACK
141a, 142a, 242a CYLINDRICAL PART
141c, 142c EDGE PART
141b, 142b HANDGRIP PART
142f, 142b BASE SURFACE
142e, 242e HOLE
144b STEP
148 STEP
149 MARK
244a SIDE WALL

## Claims

1. A package (140) for a capsule medical device (2) which retains a capsule medical device (2) including dome-shaped parts (12b, 12c) provided at two ends and a body (12a) locating between the two ends, the package (140) comprising:
a plurality of retainers (144) that are arranged at predetermined intervals such that a space in which the body of the capsule medical device (2) is exposed to allow grasp of the body of the capsule medical device (2) is formed, the retainers (144) being configured to retain the body of the capsule medical device (2) in a manner of allowing the capsule medical device (2) to be taken out from the package (140); and
a base part (1423) in which a hole (142e) into which a predetermined end (12b) of the capsule medical device (2) in a long axis (La) is fitted and mounted is formed on a main surface (142f),
wherein
the retainers (144) are configured to retain at least one part of an edge part of the body of the capsule medical device (2) in such a manner that the capsule medical device (2) can be taken out from the package (140) in a longitudinal direction (La) of the capsule medical device (2),
each of the retainers (144) is formed by a convex part protruding from the main surface (142f) of the base part uninterruptedly with a part of a side wall of the hole (142e), and
information (149) indicating a grasp direction is shown on the main surface (142f) of the base part,
whereineach of the retainers (144) has a fan like shape with a width which narrows from the outer circumference of the package (140) toward a side surface of the capsule medical device (2), and wherein an area not occupied by the retainers (144) serves as a non-retaining part (145) having a shape with a width which narrows from the outer circumference of the package (140) toward the side surface of the capsule medical device (2), and
wherein each of the retainers (144) is configured to retain the capsule medical device (2) by protruding up to a vicinity of a center of the body of the capsule medical device (2), and the non-retaining part (145) is configured to expose the body of the capsule medical device (2) to allow the grasp of the body of the capsule medical device (2).

2. The package (140) for a capsule medical device (2) according to claim 1, wherein a surface of the package (140) retaining the capsule medical device (2) is closed by a sterilization sheet (143) having sterilizing gas permeability.

3. A system comprising the package (140) according to claim 1 or 2 and the capsule medical device (2) retained therewithin.

## Patentansprüche

1. Verpackung (140) für eine medizinische Kapseleinrichtung (2), die eine medizinische Kapseleinrichtung (2) hält, welche kuppelförmige Teile (12b, 12c), die an zwei Enden vorgesehen sind, und einen Körper (12a) umfasst, der zwischen den zwei Enden angeordnet ist, wobei die Verpackung (140) umfasst:
eine Mehrzahl von Haltern (144), die in vorbestimmten Intervallen angeordnet sind, so dass ein Raum gebildet ist, in welchem der Körper der medizinischen Kapseleinrichtung (2) freigelegt ist, um das Greifen des Körpers der medizinischen Kapseleinrichtung (2) zu erlauben, wobei die Halter (144) dazu eingerichtet sind, den Körper der medizinischen Kapseleinrichtung (2) in einer Art und Weise zu halten, die es erlaubt, die medizinische Kapseleinrichtung (2) aus der Verpackung (140) zu entnehmen; und
einen Grundabschnitt (1423), in dem ein Loch (142e), in das ein vorbestimmtes Ende (12b) der medizinischen Kapseleinrichtung (2) in einer Längsachse (La) eingepasst und eingebracht ist, an einer Hauptoberfläche (142f) geformt ist,
wobei
die Halter (144) dazu eingerichtet sind, wenigstens einen Teil eines Kantenteils des Körpers der medizinischen Kapseleinrichtung (2) in so einer Art und Weise zu halten, dass die medizinische Kapseleinrichtung (2) in einer Längsrichtung (La) der medizinischen Kapseleinrichtung (2) aus der Verpackung entnommen werden kann,
jeder der Halter (144) durch einen konvexen Teil gebildet ist, der aus der Hauptoberfläche (142f) des Grundabschnitts ununterbrochen mit einem Teil einer Seitenwand des Loches (142e) vorsteht, und
Informationen (149), die eine Greifrichtung anzeigen, auf der Hauptoberfläche (142f) auf dem Grundabschnitt angezeigt werden,
wobei jeder der Halter (144) eine lüfterähnliche Form mit einer Breite hat, die sich vom Außenumfang der Verpackung (140) in Richtung einer Seitenfläche der medizinische Kapseleinrichtung (2) verjüngt, und wobei ein Bereich, der nicht von den Haltern (144) eingenommen wird, als ein nicht haltender Teil (145) dient, der eine Form mit einer Breite hat, die sich vom Außenumfang der Verpackung (140) in Richtung der Seitenfläche der medizinische Kapseleinrichtung (2) verjüngt, und
wobei jeder der Halter (144) dazu eingerichtet ist, die medizinische Kapseleinrichtung (2) zu halten, indem er bis in eine Nachbarschaft eines Zentrums der medizinischen Kapseleinrichtung (2) vorsteht, und der nicht haltende Teil (145) dazu eingerichtet ist, der Körper der medizinischen Kapseleinrichtung (2) freizuzulegen, um das Greifen des Körpers der medizinischen Kapseleinrichtung (2) zu ermöglichen.

2. Verpackung (140) für eine medizinische Kapseleinrichtung (2) gemäß Anspruch 1, wobei eine Oberfläche der Verpackung (140), die die medizinische Kapseleinrichtung (2) hält, durch eine Sterilisationsfolie geschlossen ist, die eine Permeabilität bezüglich eines sterilisierenden Gases aufweist.

3. System umfassend die Verpackung (140) gemäß Anspruch 1 oder 2 und der medizinischen Kapseleinrichtung (2), die darin gehalten ist.

## Revendications

1. Emballage (140) pour un dispositif (2) médical de type capsule qui retient un dispositif (2) médical de type capsule comprenant des parties (12b, 12c) en forme de dôme prévues au niveau des deux extrémités et un corps (12a) placé entre les deux
extrémités, l'emballage (140) comprenant :
une pluralité de dispositifs (144) de retenue qui sont disposés à des intervalles prédéterminés d'une manière telle qu'un espace dans lequel le corps du dispositif (2) médical de type capsule est exposé pour permettre la saisie du corps du dispositif (2) médical de type capsule est formé, les dispositifs (144) de retenue étant configurés
pour retenir le corps du dispositif (2) médical de type capsule de manière à permettre que le dispositif (2) médical de type capsule soit enlevé de l'emballage (140) ; et
une partie (1423) de base dans laquelle un trou (142e) dans lequel une extrémité (12b) prédéterminée du dispositif (2) médical de type capsule dans un axe longitudinal (La) est ajustée et montée est formé sur une surface principale (142f), dans lequel
les dispositifs (144) de retenue sont configurés pour retenir au moins une partie d'une partie de bord du corps du dispositif (2) médical de type capsule d'une manière telle que le dispositif (2) médical de type capsule peut être enlevé de l'emballage (140) dans une direction longitudinale (La) du dispositif (2) médical de type capsule, chacun des dispositifs (144) de retenue est formé par une partie convexe faisant saillie depuis la surface principale (142f) de la partie de base de manière ininterrompue avec une partie d'une paroi latérale du trou (142e), et
des informations (149) indiquant une direction de saisie sont représentées sur la surface principale (142f) de la partie de base,
dans lequel chacun des dispositifs (144) de retenue présente une forme d'éventail avec une largeur qui se rétrécit de la circonférence externe de l'emballage (140) à une surface latérale du dispositif (2) médical de type capsule, et dans lequel une zone n'étant pas occupée par les dispositifs (144) de retenue sert de partie (145) de non-retenue ayant une forme avec une largeur qui se rétrécit de la circonférence externe de l'emballage (140) à la surface latérale du dispositif (2) médical de type capsule, et
dans lequel chacun des dispositifs (144) de retenue est configuré pour retenir le dispositif (2) médical de type capsule en faisant saillie jusqu'au voisinage d'un centre du corps du dispositif (2) médical de type capsule, et la partie (145) de non-retenue est configurée pour exposer le corps du dispositif (2) médical de type capsule pour
permettre la saisie du corps du dispositif (2) médical de type capsule.

2. Emballage (140) pour un dispositif (2) médical de type capsule selon la revendication 1, dans lequel une surface de l'emballage (140) retenant le dispositif (2) médical de type capsule est fermée par une feuille de stérilisation (143) présentant une perméabilité au gaz de stérilisation.

3. Système comprenant l'emballage (140) selon la revendication 1 ou 2 et le dispositif (2) médical de type capsule retenu à l'intérieur.
